(19) Europäisches Patentamt
European Patent Office
Office européen des brevets

(11) Veröffentlichungsnummer: **0 069 244**
**B1**

(12) # EUROPÄISCHE PATENTSCHRIFT

(45) Veröffentlichungstag der Patentschrift: **27.12.85**

(51) Int. Cl.[4]: **A 01 N 43/90** // C07D487/08

(21) Anmeldenummer: **82105145.5**

(22) Anmeldetag: **12.06.82**

(54) **Verfahren zur Beeinflussung des Pflanzenwachstums.**

(30) Priorität: **23.06.81 DE 3124497**

(43) Veröffentlichungstag der Anmeldung:
**12.01.83 Patentblatt 83/2**

(45) Bekanntmachung des Hinweises auf die Patenterteilung:
**27.12.85 Patentblatt 85/52**

(84) Benannte Vertragsstaaten:
**BE CH DE FR IT LI NL**

(56) Entgegenhaltungen:
**EP-A-0 001 271**
**DE-A-2 615 878**

(73) Patentinhaber: **BASF Aktiengesellschaft,**
**Carl-Bosch-Strasse 38, D-6700 Ludwigshafen (DE)**

(72) Erfinder: **Schott, Eberhard Peter, Dipl.-Landwirt,**
**Oelbergstrasse 27, D-6730 Neustadt (DE)**
Erfinder: **Fischer, Volker, Dr. Dipl.-Landwirt, Alte Steige 3, D-6981 Wessental (DE)**
Erfinder: **Jung, Johann, Dr. Dipl.-Landwirt,**
**Hardenburgstrasse 19, D-6703 Limburgerhof (DE)**
Erfinder: **Rosebrock, Henning, Oberlienstrasse 15,**
**D-7022 Stetten (DE)**

EP 0 069 244 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Beeinflussung des Pflanzenwachstums, z. B. von Reis oder Zierpflanzen durch Behandlung der Samen mit einer polycyclischen stickstoffhaltigen organischen Verbindung. Bei Pflanzen, die in arider Umgebung gedeihen, hat es darüber hinaus einen günstigen Einfluß auf den Wasserhaushalt der Pflanzen.

Es ist bekannt, polycyclische stickstoffhaltige organische Verbindungen zur Beeinflussung des Pflanzenwachstums zu verwenden (DE-A-2 615 878 und EP-A-1 271). Die Aufwandmengen betragen dabei 100 g bis 15 kg Wirkstoff je ha.

Diese Aufwandmengen sind wegen der hohen Kosten der genannten polycyclischen Verbindungen wirtschaftlich nicht tragbar.

Die nachstehend im einzelnen beschriebenen Aufgaben bestehen daher nach wie vor:

Bei der Anzucht von Pflanzen aus Samen ist es üblich, die Samen in einem speziellen Anzuchtbeet, beispielsweise im Gewächshaus, auszusäen. Die Samen keimen danach und entwickeln sich zu Jungpflanzen. Diese Jungpflanzen werden später aus dem Anzuchtbeet herausgenommen und an ihren endgültigen Standort verpflanzt (Transplantation). Die Jungpflanzen dürfen bei ihrer Verpflanzung nicht zu klein aber auch nicht zu groß sein. Die Verpflanzung muß daher in einem bestimmten, durch Erfahrung ermittelten Wachstumsstadium der Jungpflanzen vorgenommen werden.

Es kommt häufig vor, daß zum Zeitpunkt der Transplantation in das Freiland, z. B. wegen ungünstiger Witterung, eine Verpflanzung nicht möglich ist. Die angezogenen Jungpflanzen wachsen weiter und sind bei einer späteren Transplantation dann schon zu groß. Solche Pflanzen sind anfälliger gegen den Streß der Verpflanzung. Auch ist eine mechanische Auspflanzung mit zu großen Pflanzen technisch nicht mehr möglich.

Die Standfestigkeit von Getreidepflanzen wie z. B. von Reis, hängt u. a. von der Halmlänge ab. Eine Verringerung derselben reduziert das entsprechende Hebelmoment und somit die Gefahr des statischen Halmbruches. Das Risiko von lagernden Getreidebeständen wird verringert.

Im Zierpflanzenbau werden kompakte Pflanzen im Vergleich zu zu großen oder zu voluminösen Pflanzen bevorzugt. So wird dadurch z. B. die Pflanzenanzahl je Einheit Stellfläche im Gewächshaus oder das Größenverhältnis Blüten : Sproß erhöht und somit die Marktqualität erhöht.

Eine weitere Aufgabe der Erfindung ergibt sich durch das Bedürfnis, das Wachstum von Pflanzen so zu beeinflussen, daß sie unter Bedingungen des Wassermangels, also in arider Umgebung, besser gedeihen und/oder weniger Wasser verbrauchen.

Eine Einsparung von Wasser dient nicht nur der Konservierung desselben in Gebieten mit Wassermangel, sondern wirkt auch der Versalzung der Bodenoberfläche durch Verdunstung entgegen.

Neben bekannten ackerbaulichen Techniken, z. B. dem bekannten Dry-Farming-System, wird auch über die Beeinflussung der Transpiration der Nutzpflanzen versucht, eine Einsparung von Wasser zu erzielen. Eine Reduzierung der Transpiration, insbesondere bei breitblättrigen Pflanzen, z. B. Weinreben, kann durch Aufsprühen von filmbildenden Stoffen (Polymere, Paraffin) erreicht werden (US-PS 3 676 102 und 3 339 373). Da bei diesem Verfahren aber die für den Gasaustausch wesentlichen Spaltöffnungen (Stomata) in ihrer Funktion gehemmt werden, sinkt häufig die Bildung von Grünmasse durch die Nutzpflanzen, weil diese in ihrem Wachstum gehindert werden. Unabhängig davon eignen sich schmalblättrige Pflanzen weniger für eine derartige Behandlung, weil ihre Blattfläche geringer ist als die breitblättriger Pflanzen und weil auf den schmalen Blättern nur wenig filmbildendes Material haften bleibt.

Es wurde nun gefunden, daß sich Wachstum und Wasserhaushalt von Pflanzen mittels einer polycyclischen stickstoffhaltigen Verbindung der Formel

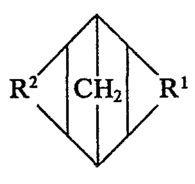

in der $R^1$ den Rest 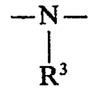 oder 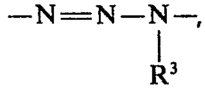, $R^2$ den Rest $-N{=}N-$ oder $-NH-NH-$

und $R^3$ den Rest

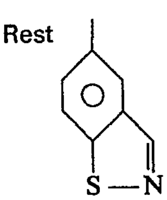

oder einen Phenylrest bedeutet, der durch Halogen, Fluoralkyl mit 1 bis 2 C-Atomen, $NO_2$ oder $C_1-C_3$-Alkyl substituiert ist, durch eine Behandlung der Samen der Pflanzen günstig beeinflussen lassen, wobei die Aufwandmenge der Behandlung so gewählt wird, daß sich für die besäte Bodenfläche eine Wirkstoffmenge von weniger als 100 g Wirkstoff je ha ergibt.

Dadurch ist es z. B. bei Reis möglich, trotz verspäteter Transplantation qualitativ hochwertige Pflanzen zu verpflanzen bzw. bei Reis die Standfestigkeit und bei Zierpflanzen die Pflanzenanzahl je Einheit Stellfläche oder das Größenverhältnis Blüten : Sproß zu erhöhen und somit die Marktqualität zu erhöhen. Überraschend ist es ferner, daß sich trotz anfänglicher Wuchshemmung die physiologische Entwicklung der Jungpflanzen nach der Verpflanzung beschleunigt. Behandelte Pflanzen sind zum Zeitpunkt der Transplantation gedrungen, kräftig und zeigen eine größere Wachstumsrate. Dieses führt nach der Transplantation gegenüber unbehandelten Pflanzen zu einem besseren Überstehen des Verpflanzungsstresses, einer höheren Widerstandsfähigkeit gegenüber ungünstigen Witterungsverhältnissen und zu einer schnelleren Entwicklung. Diese Vorteile sind auch dann von Nutzen, wenn keine verspätete Transplantation der Pflanzen erfolgt. Es können daher ganz allgemein die Samen, aus denen die Jungpflanzen wachsen sollen, erfindungsgemäß behandelt werden. Die aus behandelten Samen hervorgehenden Pflanzen erfüllen im Gegensatz zu unbehandelten folgende Anforderungen an hochwertige Qualitätspflanzen:

- kein zu schnelles Wachstum bei hohen Temperaturen vor der Transplantation,
- bessere Bewurzelung,
- geringere Kälteempfindlichkeit vor und nach der Transplantation,
- höhere Widerstandsfähigkeit gegenüber dem Transplantationsschock,
- schnellere Bewurzelung und Bestockung nach der Transplantation,
- Verzögerung des Verkümmerns (Seneszenz) der älteren Blätter und Blattscheiden,
- kompakterer Wuchs, geringeres Einzelpflanzenvolumen.

Eine weitere Wirkung wird beobachtet, wenn man z. B. bei Maissamen, die in Gebieten mit Wassermangel gezogen werden, die vorgenannte Aufwandmenge anwendet.

Die Verbesserung der Nutzung des pflanzenverfügbaren Wassers kann z. B. durch Verminderung der Transpiration aber auch durch eine Veränderung des Wurzel : Sproß-Verhältnisses erreicht werden.

Unter Verminderung der Transpiration ist die Verminderung der Wasserausscheidung in Gasform durch die Oberfläche der Nutzpflanzen zu verstehen.

Bei entsprechender Dosierung der Wirkstoffe treten keine negativen Auswirkungen auf Wachstum und Ertrag der Nutzpflanzen auf.

Die nachstehenden Beispiele illustrieren das erfindungsgemäße Verfahren:

## I. Wechselwirkung zwischen Aufwandmenge und biologischer Wirkung

### Beispiel 1

Saatgut der Reissorte »Tainung 67« wurde am 29. 11. 80 während 24 Stunden bei einer Temperatur von 30°C durch Tauchbeizung mit einer Dispersion des Wirkstoffs in Wasser behandelt. Die Saat in Anzuchtkästen erfolgte am 05. 12. 80. Am 07. 12. 80 lief die Saat auf. 13 und 21 Tage nach der Saat wurde die Wuchshöhe und Wachstumsrate ermittelt. Folgende Daten wurden gewonnen:

| | Tage nach der Saat | Wirkstoff g/ha (Konzentration des Wirkstoffs in der Beizflüssigkeit in ppm) | | |
|---|---|---|---|---|
| | | Unbehandelt | Substanz A | |
| | | 0 | 0,5 (5) | 1,0 (10) |
| Wuchshöhe, cm | 13 | 8,5 | 6,9 | 6,4 |
| Wuchshöhe, cm | 21 | 9,0 | 8,0 | 7,5 |
| Wachstumsrate, % | 21 | 5,9 [(9,0–8,5): 8,5 × 100 = 5,9%] | 15,9 | 17,2 |

Substanz A = 5-(4-Chlorphenyl)-3,4,5,9,10-pentaazatetracyclo-[5,4,1,$0^{2,6}$,$0^{8,11}$]-dodeca-3,9-dien.

Beispiel 2

Saatgut von Reis der Sorte »Kaohshing 141« wurde am 29. 11. 80 während 24 Stunden bei einer Temperatur von 30°C durch Tauchbeizung behandelt. Die Saat in Anzuchtkästen erfolgte am 05. 12. 80. Der Auflauf war am 07. 12. 80. 13 und 21 Tage nach der Saat wurde die Wuchshöhe und Wachstumsrate ermittelt. Folgende Daten wurden gewonnen:

| | Tage nach der Saat | Wirkstoff g/ha (ppm) | | |
|---|---|---|---|---|
| | | Unbehandelt | Substanz A | |
| | | 0 | 0,5 (5) | 1,0 (10) |
| Wuchshöhe, cm | 13 | 10,5 | 8,5 | 7,0 |
| Wuchshöhe, cm | 21 | 11,2 | 10,2 | 9,5 |
| Wachstumsrate, % | 21 | 6,7 | 20,0 | 35,7 |

Beispiel 3

Saatgut von Reis der Sorte »Tainan 5« wurde am 18. 04. 80 während 24 Stunden bei einer Temperatur von 30°C durch Tauchbeizung behandelt. Die Saat in Anzuchtkästen erfolgte am 23. 04. 80. Am 25. 04. 80 lief die Saat auf. 13 und 21 Tage nach der Saat wurde die Wuchshöhe gemesssen. Folgende Daten wurden gewonnen:

| | Tage nach der Saat | Wirkstoff g/ha (ppm) | | | |
|---|---|---|---|---|---|
| | | Unbehandelt | Substanz A | | |
| | | 0 | 0,25 (2,5) | 0,5 (5,0) | 1,0 (10,0) |
| Wuchshöhe, cm | 13 | 9,2 | 6,9 | 5,8 | 5,3 |
| Wuchshöhe, cm | 21 | 10,1 | 9,0 | 8,5 | 7,9 |
| Wachstumsrate, % | 21 | 9,8 | 30,4 | 46,6 | 49,1 |

Beispiel 4

Saatgut von Reis der Sorte »Inabawase« wurde am 27. 04. 80 während 24 Stunden bei einer Temperatur von 30°C durch Tauchbeizung behandelt. Der Auflauf wurde am 30. 04. 80 beobachtet. Die Saat in Anzuchtkästen erfolgte am 28. 04. 80. 7, 9, 13, 19 und 28 Tage nach der Saat wurde die Wuchshöhe und Wachstumsrate sowie 28 Tage nach der Saat das Blatt- und Wurzelgewicht je Pflanze ermittelt. Folgende Ergebnisse wurden gewonnen:

| | Tage nach der Saat | Wirkstoff g/ha (ppm) | | |
|---|---|---|---|---|
| | | Unbehandelt | Substanz A | |
| | | 0 | 1 (10) | 10 (100) |
| Wuchshöhe, cm | 7 | 6,0 | 3,4 | 1,6 |
| Wuchshöhe, cm | 9 | 6,0 | 4,4 | 2,8 |
| Wachstumsrate, % | 9 | 0,0 | 29,4 | 75,0 |

Fortsetzung

| | Tage nach der Saat | Wirkstoff g/ha (ppm) | | |
|---|---|---|---|---|
| | | Unbehandelt | Substanz A | |
| | | 0 | 1 (10) | 10 (100) |
| Wuchshöhe, cm | 13 | 8,2 | 6,5 | 3,8 |
| Wachstumsrate, % | 13 | 36,7 | 47,7 | 35,7 |
| Wuchshöhe, cm | 19 | 15,5 | 11,0 | 7,5 |
| Wachstumsrate, % | 19 | 89,0 | 69,2 | 97,4 |
| Wuchshöhe, cm | 28 | 15,8 | 12,0 | 7,8 |

| | Tage nach der Saat | Wirkstoff g/ha (ppm) | | |
|---|---|---|---|---|
| | | Unbehandelt | Substanz A | |
| | | 0 | 1 (10) | 10 (100) |
| Wachstumsrate, % | 28 | 1,9 | 9,1 | 4,0 |
| Blattgewicht TM, mg/Pflanze | 28 | 1,11 | 0,97 | 0,62 |
| Wurzelgewicht TM | | | | |
| Wurzelgewicht TM, mg/Pflanze | 28 | 1,16 | 1,11 | 1,0 |
| Wurzel: Blattgewicht TM | 28 | 1,04 : 1 | 1,14 : 1 | 1,61 : 1 |

TM = Trockenmasse.

Den Beispielen 1 bis 4 kann entnommen werden, daß unabhängig von der Sorte Aufwandmengen der Substanz A in einer Größenordnung von 0,0125 g/ha (= 0,05 ppm) bis 100 g/ha (= 1000 ppm) zu einer deutlichen Hemmung des Streckungswachstums führen. Überraschend ist, daß schon 0,0125 g/ha (= 0,05 ppm) 13 Tage nach der Behandlung zu einer Wachstumshemmung von 19,0% führen.

Der anfänglichen Wuchshemmung nach der Saatgutbeizung folgt gegenüber unbehandelten Pflanzen eine deutlich gesteigerte Wachstumsrate. Diese steht in Zusammenhang mit einem erheblich erweiterten Wurzel : Blatt-Gewichtsverhältnis von 1,14—1,61 : 1 gegenüber etwa 1 : 1 bei unbehandelten Reispflanzen.

Die Substanz A ermöglicht somit durch Saatguttauchbeizung mit Aufwandmengen von 0,25 g/ha (= 2,5 ppm) bis 100 g/ha (= 1000 ppm) eine kontrollierte Jungpflanzenanzucht. Solche behandelten Jungpflanzen sind zum Zeitpunkt der Transplantation in das Freiland robuster und widerstandsfähiger. Nach der Verpflanzung entwickeln sie sich schneller als Reispflanzen aus unbehandeltem Saatgut.

Beispiel 5

Am 28. 04. 80 wurde Saatgut der Reissorte »Nihonbare« für 24 Stunden bei einer Temperatur von 30°C im Tauchbad mit der Substanz A gebeizt. Die Saat in Anzuchtkästen erfolgte am 04. 05. 80. Am 06. 05. 80 lief die Saat auf. Die Transplantation in das Freiland erfolgte am 28. 05. 80. 19 Tage nach der Saat bzw. 16 und 22 Tage nach der Transplantation wurde die Wuchshöhe gemessen. Die Anzahl der Bestockungstriebe wurde am 16. und 22. Tag nach der Transplantation ausgezählt. Ebenfalls am 22. Tag nach der Transplantation wurden die Wurzellänge/Pflanze sowie das Blatt- und Wurzelgewicht je Pflanze festgestellt.

Dieses Versuchsbeispiel zeigt, daß die mit der Substanz A über das Saatgut behandelten Reispflanzen zum Zeitpunkt der Transplantation in das Freiland die ideale Wuchshöhe von etwa 13 cm erreicht haben, die unbehandelten Pflanzen jedoch zu diesem Zeitpunkt dieses Maß schon weit überschritten hatten und als Pflanzgut unbrauchbar waren.

16 und 22 Tage nach der Transplantation haben die behandelten Reispflanzen die Wuchshöhe der unbehandelten Pflanzen erreicht und überschritten.

| | Tage nach der Saat | Wirkstoff g/ha (ppm) | | | |
|---|---|---|---|---|---|
| | | Unbehandelt | Substanz A | | |
| | | 0 | 0,5 (5) | 0,75 (7,5) | 1,0 (10) |
| Wuchshöhe, cm | 19 | 19,3 | 12,7 | 10,5 | 9,7 |
| Wuchshöhe, cm | 40 | 40,0 | 37,3 | 39,3 | 43,5 |
| Wachstumsrate, % | 40 | 107,2 | 193,7 | 274,3 | 348,4 |
| Wuchshöhe, cm | 46 | 46,7 | 46,5 | 47,2 | 54,2 |
| Wachstumsrate, % | 46 | 16,8 | 24,7 | 20,1 | 24,6 |
| Bestockungstriebe, St./Pflanze | 40 | 3,2 | 3,7 | 4,3 | 4,3 |
| Bestockungstriebe, St./Pflanze | 46 | 6,0 | 7,5 | 9,2 | 9,2 |
| Wurzellänge, mm/Pflanze | 46 | 170,8 | 162,5 | 176,8 | 200,8 |
| Blattgewicht TM, mg/Pflanze | 46 | 84,5 | 84,5 | 99,5 | 109,0 |
| Wurzelgewicht TM, mg/Pflanze | 46 | 33,5 | 31,0 | 34,5 | 43,5 |
| Wurzel: Blattgewicht, TM | 46 | 0,40 : 1 | 0,37 : 1 | 0,35 : 1 | 0,40 : 1 |

Diese Entwicklungsbeschleunigung nach der Transplantation zeigen die behandelten Pflanzen auch hinsichtlich der Anzahl der Bestockungstriebe, der Wurzellänge und der Trockenmassegewichte.

Mit der Substanz A behandelte Reispflanzen erreichen den Zeitpunkt der Transplantation in einem gedrungenen und kräftigen Zustand. Sie überstehen den Streß der Transplantation in das Freiland besser und entwickeln sich anschließend schneller.

## II. Wechselwirkung zwischen Anwendungsmethode und biologischer Wirkung

### Beispiel 6

Saatgut von Reis der Sorte »Inabawase« wurde am 27. 04. 78 während 24 Stunden bei einer Temperatur von 30°C durch Tauchbeizung behandelt. Die Saat in Anzuchtkästen erfolgte am 28. 04. 78. Am 30. 04. 78 wurde der Auflauf beobachtet. 7, 9, 13, 19 und 28 Tage nach der Saat wurde die Wuchshöhe und Wachstumsrate sowie am 28. Tag nach der Saat das Blatt- und Wurzelgewicht je Pflanze ermittelt. Folgende Resultate wurden gewonnen:

| | Tage nach der Saat | Wirkstoff g/ha (ppm) | | | | |
|---|---|---|---|---|---|---|
| | | Unbehandelt | Substanz A | | Substanz B | |
| | | 0 | 1 (10) | 10 (100) | 1 (10) | 10 (100) |
| Wuchshöhe, cm | 7 | 6,0 | 3,4 | 1,6 | 3,9 | 2,2 |
| Wuchshöhe, cm | 9 | 6,0 | 4,4 | 2,8 | 5,0 | 3,2 |
| Wachstumsrate, % | 9 | 0,0 | 29,4 | 75,0 | 28,2 | 45,4 |

Fortsetzung

| | Tage nach der Saat | Wirkstoff g/ha (ppm) | | | | |
|---|---|---|---|---|---|---|
| | | Unbe-handelt | Substanz A | | Substanz B | |
| | | 0 | 1 (10) | 10 (100) | 1 (10) | 10 (100) |
| Wuchshöhe, cm | 13 | 8,2 | 6,5 | 3,8 | 7,2 | 4,8 |
| Wachstumsrate, % | 13 | 36,7 | 47,7 | 35,7 | 44,0 | 50,0 |
| Wuchshöhe, cm | 19 | 15,5 | 11,0 | 7,5 | 12,2 | 8,8 |
| Wachstumsrate, % | 19 | 89,0 | 69,2 | 97,4 | 69,4 | 83,3 |
| Wuchshöhe, cm | 28 | 15,8 | 12,0 | 7,8 | 12,2 | 8,8 |
| Wachstumsrate, % | 28 | 1,9 | 9,1 | 4,0 | 0 | 0 |
| Blattgewicht TM, mg/Pflanze | 28 | 1,11 | 0,97 | 0,62 | 0,89 | 0,78 |
| Wurzelgewicht TM, mg/Pflanze | 28 | 1,16 | 1,11 | 1,0 | 1,2 | 1,13 |
| Wurzel : Blattgewicht, TM | 28 | 1,04 : 1 | 1,14 : 1 | 1,16 : 1 | 1,35 : 1 | 1,45 : 1 |

Substanz B = 5-(4-Bromphenyl)-3,4,5,9,10-pentaazatetracyclo-[$5,4,1,0^2,0^{2,11}$]-dodeca-3,9-dien.

Beispiel 7

Am 27. 04. 78 wurden die Substanzen A und B als wäßrige Dispersion (Slurry) zur Beizung auf das Saatgut der Reissorte »Inabawase« aufgetragen. Die Saat in die Anzuchtkästen erfolgte ebenfalls am 27. 04. 78. Am 29. 04. 78 lief die Saat auf. 8, 10, 14, 20 und 29 Tage nach der Saat wurde die Wuchshöhe und Wachstumsrate sowie 29 Tage nach der Saat das Blatt- und Wurzelgewicht je Pflanze ermittelt. Folgende Versuchsergebnisse wurden gefunden:

| | Tage nach der Saat | Wirkstoff g/ha (ppm) | | | | |
|---|---|---|---|---|---|---|
| | | Unbe-handelt | Substanz A | | Substanz B | |
| | | 0 | 1 (10) | 10 (100) | 1 (10) | 10 (100) |
| Wuchshöhe, cm | 8 | 5,6 | 1,8 | 0,5 | 2,8 | 0,8 |
| Wuchshöhe, cm | 10 | 6,8 | 2,2 | 0,9 | 3,4 | 1,4 |
| Wachstumsrate, % | 10 | 21,4 | 22,2 | 80,0 | 21,4 | 75,0 |
| Wuchshöhe, cm | 14 | 9,1 | 2,6 | 1,1 | 5,0 | 1,9 |
| Wachstumsrate, % | 14 | 33,8 | 18,2 | 22,2 | 47,0 | 35,7 |
| Wuchshöhe, cm | 20 | 16,2 | 4,2 | 2,4 | 8,5 | 2,8 |
| Wachstumsrate, % | 20 | 78,0 | 61,5 | 118,2 | 70,0 | 47,4 |
| Wuchshöhe, cm | 29 | 17,5 | 5,8 | 3,1 | 9,8 | 3,5 |
| Wachstumsrate, % | 29 | 8,0 | 38,1 | 29,2 | 1,53 | 25,0 |

Fortsetzung

| | Tage nach der Saat | Wirkstoff g/ha (ppm) Unbehandelt | Substanz A | | Substanz B | |
|---|---|---|---|---|---|---|
| | | 0 | 1 (10) | 10 (100) | 1 (10) | 10 (100) |
| Blattgewicht TM, mg/Pflanze | 29 | 1,13 | 0,76 | 0,42 | 0,81 | 0,31 |
| Wurzelgewicht TM, mg/Pflanze | 29 | 1,4 | 1,36 | 1,14 | 1,3 | 1,1 |
| Wurzel : Blattgewicht, TM | 29 | 1,24 : 1 | 1,79 : 1 | 2,71 : 1 | 1,6 : 1 | 3,55 : 1 |

Die Beispiele belegen, daß die Substanzen A und B das Streckungswachstum der Pflanzen deutlich hemmen. Dabei spielt es keine wesentliche Rolle, welche Behandlungsmethode zur Anwendung der Substanzen A und B gewählt wird. Folgende Methoden können beispielsweise angewendet werden:

– Saatguttauchbeizung (Beispiel 6);
– Slurrybeizung (Beispiel 7).

Die anfänglich gewünschte Wuchshemmung ab dem Auflauf bis zum Zeitpunkt der Transplantation der Reispflanzen in das Freiland wird ab der 2. Woche abgelöst durch eine Stimulation des Wachstums. Diese nimmt mit steigender Aufwandmenge zu. Dieses Phänomen setzt sich auch noch bis nach der Transplantation fort, so daß die behandelten Reispflanzen sich im Freiland schneller entwickeln.

Dieses Abklingen der ursprünglich gewünschten Wuchshemmung und der Wechsel zu einer gewünschten späteren schnelleren Entwicklung wird durch das Wurzel : Blatt-Verhältnis der aus behandelten Samen hervorgegangenen Pflanzen mitverursacht. Gegenüber unbehandelten Pflanzen mit einem Verhältnis von 0,98 : 1 bis 1,12 : 1 zeigen die behandelten Sämlinge etwa 4 Wochen nach der Anwendung der Substanz A ein solches von 1,08 : 1 bis zu mehr als 124 : 1 und nach der Substanz B ein solches von 1,07 : 1 bis zu 23,0 : 1.

III. Vergleich von Bioregulatoren mit verschiedener chemischer Struktur

Beispiele 8–10

Saatgut von Reis der Sorte »Inabawase« wurde am 27. 04. 78 während 24 Stunden bei einer Temperatur von 30°C durch Tauchbeizung behandelt. Am 28. 04. 78 erfolgte die Saat in die Anzuchtkästen. Am 30. 04. 78 lief die Saat auf. 7, 9, 13, 19 und 28 Tage nach der Saat wurde die Wuchshöhe und Wachstumsrate sowie 28 Tage nach der Saat das Blatt- und Wurzelgewicht je Pflanze ermittelt. Nachstehende Vergleiche wurden angestellt:

Beispiel 8

| | Tage nach der Saat | Wirkstoff g/ha (ppm) Unbehandelt | Substanz A | | Substanz C |
|---|---|---|---|---|---|
| | | 0 | 1 (10) | 10 (100) | 10 (100) |
| Wuchshöhe, cm | 7 | 6,0 | 3,4 | 1,6 | 7,0 |
| Wuchshöhe, cm | 9 | 6,0 | 4,4 | 2,8 | 7,8 |
| Wachstumsrate, % | 9 | 0,0 | 29,4 | 75,0 | 11,1 |
| Wuchshöhe, cm | 13 | 8,2 | 6,5 | 3,8 | 11,8 |
| Wachstumsrate, % | 13 | 36,7 | 47,7 | 35,7 | 51,3 |

Fortsetzung

| | Tage nach der Saat | Wirkstoff g/ha (ppm) | | | |
|---|---|---|---|---|---|
| | | Unbehandelt | Substanz A | | Substanz C |
| | | 0 | 1 (10) | 10 (100) | 10 (100) |
| Wuchshöhe, cm | 19 | 15,5 | 11,0 | 7,5 | 16,8 |
| Wachstumsrate, % | 19 | 89,0 | 69,2 | 97,4 | 42,3 |
| Wuchshöhe, cm | 28 | 15,8 | 12,0 | 7,8 | 17,0 |
| Wachstumsrate, % | 28 | 1,9 | 9,1 | 4,0 | 1,2 |
| Blattgewicht TM, mg/Pflanze | 28 | 1,11 | 0,97 | 0,62 | 1,22 |
| Wurzelgewicht TM, mg/Pflanze | 28 | 1,16 | 1,11 | 1,0 | 1,17 |
| Wurzel : Blattgewicht, TM | 28 | 1,04 : 1 | 1,14 : 1 | 1,61 : 1 | 0,96 : 1 |

Substanz C = 2-Chlorethyl-phosphonsäure.

Beispiel 9

| | Tage nach der Saat | Wirkstoff g/ha (ppm) | | | |
|---|---|---|---|---|---|
| | | Unbehandelt | Substanz A | | Substanz D |
| | | 0 | 1 (10) | 10 (100) | 10 (100) |
| Wuchshöhe, cm | 7 | 6,0 | 3,4 | 1,6 | 6,6 |
| Wuchshöhe, cm | 9 | 6,0 | 4,4 | 2,8 | 8,0 |
| Wachstumsrate, % | 9 | 0,0 | 29,4 | 75,0 | 21,2 |
| Wuchshöhe, cm | 13 | 8,2 | 6,5 | 3,8 | 11,2 |
| Wachstumsrate, % | 13 | 36,7 | 47,7 | 35,7 | 40,0 |
| Wuchshöhe, cm | 19 | 15,5 | 11,0 | 7,5 | 15,5 |
| Wachstumsrate, % | 19 | 89,0 | 69,2 | 97,4 | 38,4 |
| Wuchshöhe, cm | 28 | 15,8 | 12,0 | 7,8 | 15,5 |
| Wachstumsrate, % | 28 | 1,9 | 9,1 | 4,0 | 0,0 |
| Blattgewicht TM, mg/Pflanze | 28 | 1,11 | 0,97 | 0,62 | 1,04 |
| Wurzelgewicht TM, mg/Pflanze | 28 | 1,16 | 1,11 | 1,0 | 1,17 |
| Wurzel : Blattgewicht, TM | 28 | 1,04 : 1 | 1,14 : 1 | 1,61 : 1 | 1,12 : 1 |

Substanz D = 1,1-Dimethyl-piperidinium-chlorid.

Beispiel 10

| | Tage nach der Saat | Wirkstoff g/ha (ppm) | | | |
|---|---|---|---|---|---|
| | | Unbehandelt | Substanz A | | Substanz E |
| | | 0 | 1 (10) | 10 (100) | 10 (100) |
| Wuchshöhe, cm | 7 | 6,0 | 3,4 | 1,6 | 6,8 |
| Wuchshöhe, cm | 9 | 6,0 | 4,4 | 2,8 | 8,1 |
| Wachstumsrate, % | 9 | 0,0 | 29,4 | 75,0 | 19,1 |
| Wuchshöhe, cm | 13 | 8,2 | 6,5 | 3,8 | 11,2 |
| Wachstumsrate, % | 13 | 36,7 | 47,7 | 35,7 | 38,3 |
| Wuchshöhe, cm | 19 | 15,5 | 11,0 | 7,5 | 16,0 |
| Wachstumsrate, % | 19 | 89,0 | 69,2 | 97,4 | 42,8 |
| Wuchshöhe, cm | 28 | 15,8 | 12,0 | 7,8 | 16,5 |
| Wachstumsrate, % | 28 | 1,9 | 9,1 | 4,0 | 3,1 |
| Blattgewicht TM, mg/Pflanze | 28 | 1,11 | 0,97 | 0,62 | 1,19 |
| Wurzelgewicht TM, mg/Pflanze | 28 | 1,16 | 1,11 | 1,00 | 1,24 |
| Wurzel : Blattgewicht, TM | 28 | 1,04 : 1 | 1,14 : 1 | 1,61 : 1 | 1,04 : 1 |

Substanz E = Mischung aus 2 Teilen (Gew.-Teile) 1,1-Dimethyl-piperidinium-chlorid und 1 Teil 2-Chlorethyl-phosphonsäure.

Den Beispielen 8 bis 10 kann entnommen werden, daß im Gegensatz zu Substanz A keine der drei vergleichsweise geprüften Substanzen C, D und E eine Hemmung des Streckungswachstums der jungen Reispflanzen bewirkt. Eine allgemeine Wachstumsstimulation ist gegenüber unbehandelten Pflanzen zu beobachten.

Auch im Wurzel : Blattgewicht-Verhältnis, bezogen auf die Trockenmasse, unterscheiden sich die mit den Substanzen C, D und E behandelten Pflanzen gegenüber unbehandelten kaum.

Beispiele 11 bis 13

Am 27. 04. 78 wurde Saatgut der Reissorte »Inabawase« nach dem »Slurry«-Beizverfahren mit den Wirkstoffen der Substanzen A, C, D und E behandelt. Die Saat in die Anzuchtkästen erfolgte ebenfalls am 27. 04. 78. Am 29. 04. 78 lief die Saat auf. 8, 10, 14, 20 und 28 Tage nach der Saat wurde die Wuchshöhe und Wachstumsrate sowie 29 Tage nach der Saat das Blatt- und Wurzelgewicht je Pflanze ermittelt.

Auch hier kann festgestellt werden, daß im Gegensatz zu der Substanz A keine der vergleichsweise geprüften Substanzen C, D und E eine Hemmung des Längenwachstums der jungen Reispflanzen verursachte. Es wurden ähnliche Ergebnisse wie in den Versuchsbeispielen 8 bis 10 gefunden.

Beispiel 11

| | Tage nach der Saat | Wirkstoff g/ha (ppm) | | | |
|---|---|---|---|---|---|
| | | Unbehandelt | Substanz A | | Substanz C |
| | | 0 | 1 (10) | 10 (100) | 10 (100) |
| Wuchshöhe, cm | 8 | 5,6 | 1,8 | 0,5 | 6,2 |
| Wuchshöhe, cm | 10 | 6,8 | 2,2 | 0,9 | 8,1 |
| Wachstumsrate, % | 10 | 21,4 | 22,2 | 80,0 | 30,6 |
| Wuchshöhe, cm | 14 | 9,1 | 2,6 | 1,1 | 10,6 |
| Wachstumsrate, % | 14 | 33,8 | 18,2 | 22,2 | 30,9 |
| Wuchshöhe, cm | 20 | 16,2 | 4,2 | 2,4 | 16,2 |
| Wachstumsrate, % | 20 | 78,0 | 61,5 | 118,2 | 52,8 |
| Wuchshöhe, cm | 29 | 17,5 | 5,8 | 3,1 | 17,0 |
| Wachstumsrate, % | 29 | 8,0 | 38,1 | 29,2 | 4,9 |
| Blattgewicht TM, mg/Pflanze | 29 | 1,13 | 0,76 | 0,42 | 1,13 |
| Wurzelgewicht TM, mg/Pflanze | 29 | 1,40 | 1,36 | 1,14 | 1,40 |
| Wurzel : Blattgewicht, TM | 29 | 1,24 : 1 | 1,79 : 1 | 2,71 : 1 | 1,24 : 1 |

Beispiel 12

| | Tage nach der Saat | Wirkstoff g/ha (ppm) | | | |
|---|---|---|---|---|---|
| | | Unbehandelt | Substanz A | | Substanz D |
| | | 0 | 1 (10) | 10 (100) | 10 (100) |
| Wuchshöhe, cm | 8 | 5,6 | 1,8 | 0,5 | 6,5 |
| Wuchshöhe, cm | 10 | 6,8 | 2,2 | 0,9 | 7,8 |
| Wachstumsrate, % | 10 | 21,4 | 22,2 | 80,0 | 20,0 |
| Wuchshöhe, cm | 14 | 9,1 | 2,6 | 1,1 | 10,8 |
| Wachstumsrate, % | 14 | 33,8 | 18,2 | 22,2 | 38,5 |
| Wuchshöhe, cm | 20 | 16,2 | 4,2 | 2,4 | 16,5 |
| Wachstumsrate, % | 20 | 78,0 | 61,5 | 118,2 | 52,8 |
| Wuchshöhe, cm | 29 | 17,5 | 5,8 | 3,1 | 17,0 |
| Wachstumsrate, % | 29 | 8,0 | 38,1 | 29,2 | 3,0 |
| Blattgewicht TM, mg/Pflanze | 29 | 1,13 | 0,76 | 0,42 | 1,72 |
| Wurzelgewicht TM, mg/Pflanze | 29 | 1,40 | 1,36 | 1,14 | 1,44 |
| Wurzel : Blattgewicht, TM | 29 | 12,4 : 1 | 1,79 : 1 | 2,71 : 1 | 0,83 : 1 |

Beispiel 13

| | Tage nach der Saat | Wirkstoff g/ha (ppm) | | | |
|---|---|---|---|---|---|
| | | Unbehandelt | Substanz A | | Substanz D |
| | | 0 | 1 (10) | 10 (100) | 10 (100) |
| Wuchshöhe, cm | 8 | 5,6 | 1,8 | 0,5 | 7,1 |
| Wuchshöhe, cm | 10 | 6,8 | 2,2 | 0,9 | 9,0 |
| Wachstumsrate, % | 10 | 21,4 | 22,2 | 80,0 | 26,8 |
| Wuchshöhe, cm | 14 | 9,1 | 2,6 | 1,1 | 11,9 |
| Wachstumsrate, % | 14 | 33,8 | 18,2 | 22,2 | 32,2 |
| Wuchshöhe, cm | 20 | 16,2 | 4,2 | 2,4 | 16,2 |
| Wachstumsrate, % | 20 | 78,0 | 61,5 | 118,2 | 36,1 |
| Wuchshöhe, cm | 29 | 17,5 | 5,8 | 3,1 | 16,8 |
| Wachstumsrate, % | 29 | 8,0 | 38,1 | 29,2 | 3,7 |
| Blattgewicht TM, mg/Pflanze | 29 | 1,13 | 0,76 | 0,42 | 1,40 |
| Wurzelgewicht TM, mg/Pflanze | 29 | 1,40 | 1,36 | 1,14 | 1,26 |
| Wurzel : Blattgewicht, TM | 29 | 1,24 : 1 | 1,79 : 1 | 2,71 : 1 | 0,9 : 1 |

Beispiel 14

Saatgut der Maissorte 3369A wurde in Form einer Naßbeize mit dem Wirkstoff A (= 5-(4-Chlorphenyl)-3,4,5,9,10-pentaazatetracyclo-[5,4,1,$0^{2,6}$,$0^{8,11}$]-dodeca-3,9-dien) behandelt, in das Freiland ausgesät und unter Wassermangel angezogen. 37 Tage nach der Saat wurden die Welkesymptome und 50 Tage nach der Saat das Blattwasserpotential mittels einer Pressure bomb festgestellt.

Die Welkesymptome wurden durch die Behandlung deutlich verringert und das Wasserpotential im Blatt erhöht.

Ein Einfluß auf die Pflanzenpopulation sowie auf die Anzahl an Kolben je Pflanze wurde nicht festgestellt.

Hingegen wurde durch die verbesserte Wasserausnutzung die Anzahl an Körnern je Kolben, das 1000-Korngewicht und der Kornertrag je Hektar klar erhöht.

| *) berechnet auf Trockenmasse | Wirkstoff A g/ha | | | |
|---|---|---|---|---|
| | 0 | 0,21 | 2,1 | 21,0 |
| Körner/Kolben*) | | | | |
| g | 74,9 | 87,1 | 99,8 | 98,9 |
| % | 100 | 116 | 133 | 132 |
| 1000-Korngewicht*) | | | | |
| g | 232 | 235 | 242 | 250 |
| % | 100 | 101 | 104 | 108 |

Fortsetzung

| *) berechnet auf Trockenmasse | Wirkstoff A g/ha | | | |
|---|---|---|---|---|
| | 0 | 0,21 | 2,1 | 21,0 |
| Kornertrag*) | | | | |
| dt/ha | 50,6 | 58,7 | 57,9 | 56,1 |
| % | 100 | 116 | 114 | 111 |
| welke Blätter, % | 50 | 51 | 38 | 14 |
| Blattwasserpotential, bar | 16,7 | – | – | 14,5 |

Beispiel 15

Saatgut der Maissorte 3369A wurde in Form einer Naßbeize mit dem Wirkstoff A behandelt, in das Freiland ausgesät und unter Wassermangel angezogen. 31 Tage nach der Saat wurden die Welkesymptome und die Wuchshöhe dieser Pflanzen bewertet.

Analog Beispiel 1 wurden auch hier die Welkesymptome der behandelten Pflanzen im Vergleich zu unbehandelten Pflanzen deutlich verringert. Eine erhebliche Verbesserung der Wassernutzung ist auch hier feststellbar. Die Behandlungen führten außerdem zu einer Hemmung des Längenwuchses zwischen 10 und 20%.

| | Wirkstoff A g/ha | | | | | |
|---|---|---|---|---|---|---|
| | 0 | 2,1 | 4,2 | 6,3 | 8,4 | 10,5 |
| welke Blätter, % | 83,1 | 13,8 | 16,7 | 15,8 | 11,7 | 7,9 |
| Wachshemmung, % | 0 | 9,3 | 9,9 | 13,9 | 18,6 | 20,1 |

Beispiel 16

Saatgut der Maissorte 3369A wurde während 2 Stunden bei einer Temperatur von 25°C durch Tauchbeizung mit einer Dispersion des Wirkstoffs A in Wasser behandelt. Eine Partie des gleichen Saatgutes wurde mit reinem Wasser gleichermaßen behandelt.

Danach erfolgte die Aussaat in das Freiland. Der Bestand wurde unter Wassermangel angezogen.

Die Auswertungen der Ertragsparameter erfolgte zur Reife der Körner. Auch bei diesem Beispiel konnten die Ertragsfaktoren deutlich verbessert werden.

Auffallend ist, daß die behandelten Pflanzen auf Grund ihres besseren Wasserstatus' gegenüber Krankheiten, hier Ustilago scitaminea, widerstandsfähiger sind. Der Anteil gesunder Maiskolben wurde durch die Behandlung mit dem Wirkstoff A erhöht.

| | Wirkstoff g/ha | | | | | | |
|---|---|---|---|---|---|---|---|
| | 0 | $H_2O$ 0 | 0,1 | 0,3 | 0,9 | 2,7 | 27,0 |
| Kolbenertrag | | | | | | | |
| dt/ha | 59,0 | 62,0 | 63,0 | 69,0 | 66,0 | 72,0 | 72,0 |
| % | 100 | 105 | 107 | 117 | 112 | 122 | 122 |

Fortsetzung

| | Wirkstoff g/ha | | | | | | |
|---|---|---|---|---|---|---|---|
| | 0 | $H_2O$ 0 | 0,1 | 0,3 | 0,9 | 2,7 | 27,0 |
| Ertrag gesunder Kolben | | | | | | | |
| dt/ha | 55,5 | 57,8 | 59,0 | 66,3 | 62,7 | 67,2 | 69,0 |
| % | 100 | 104 | 106 | 119 | 113 | 121 | 124 |
| 1000-Korngewicht | | | | | | | |
| g | 268 | 287 | 281 | 290 | 286 | 294 | 312 |
| % | 100 | 107 | 105 | 108 | 107 | 110 | 116 |

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäure, Naphthalinsulfonsäure, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rhizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Die Formulierungen enthalten zwischen 0,1 und 95 Gew.-% Wirkstoff, vorzugsweise zwischen 0,5 und 90 Gew.-%.

In verschiedenen Fällen kann sich die Kombination bzw. Mischung mit anderen wachstumsregulierenden Wirkstoffen als vorteilhaft erweisen, wie u. a. mit äthylenbildenden Stoffen verschiedener chemischer Struktur (z. B. Phosphonsäurederivate und Silane, Äthylhydrazine), Oniumverbindungen (z. B. Trimethylammonium-, Hydryzonium- und Sulfoniumsalze, Derivate von Morpholinium-, Piperidinium- und Pyridaziniumverbindungen). Von Interesse sind auch weitere wachstumsregulierende Substanzen, u. a. aus der Gruppe der Trifluormethylsulfonamido-p-acetotoluidide, Maleinsäurehydrazid, Abscissinsäurederivate, Triazolverbindungen chlorierte Phenoxyfettsäuren mit auxinähnlicher Wirkung sowie höherwertige Alkohole und Fettsäureester mit spezifischer Wirkung auf meristematische Gewebepartien.

Die erfindungsgemäßen Mittel können in ihrer angewandten Menge schwanken. Die angewandte Menge hängt hauptsächlich von der Art des gewünschten Effektes ab.

Die Aufwandmenge liegt im allgemeinen zwischen 0,01 und 99 g/ha, vorzugsweise zwischen 0,01 und 30 g Wirkstoff pro Hektar, wobei im Falle der Saatgutbezeichnung eine Umrechnung stattfindet, derart, daß die zur Beizung verwendete Menge die für die zu besäende Fläche benötigte Menge ergibt.

**Patentansprüche**

1. Verfahren zur Regulierung des Pflanzenwachstums und/oder zur Verbesserung der Nutzung des verfügbaren Wassers durch Pflanzen, dadurch gekennzeichnet, daß man die Samen der Pflanzen behandelt mit einem polycyclischen stickstoffhaltigen Wirkstoff der Formel

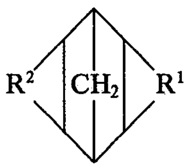

in der $R^1$ den Rest $-N-$ oder $-N{=}N-N-$, $R^2$ den Rest $-N{=}N-$ oder $-NH-NH-$

$R^3$ $R^3$

und $R^3$ den Rest

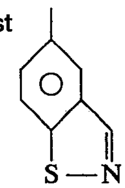

oder einen Phenylrest bedeutet, der durch Halogen, Fluoralkyl mit 1 bis 2 C-Atomen, $NO_2$ oder $C_1-C_3$-Alkyl substituiert ist, bedeutet, wobei die Aufwandmenge der Behandlung so gewählt wird, daß sich für die besäte Bodenfläche eine Wirkstoffmenge von weniger als 100 g Wirkstoff je ha ergibt.

2. Verfahren nach Anspruch 1, dadurch gekennzeichnet, daß $R^3$ 4-Chlorphenyl oder 4-Bromphenyl bedeutet.

**Claims**

1. A process for regulating plant growth and/or for improving the utilization of the available water by plants, wherein the seeds of the plants are treated with a polycyclic, nitrogen-containing active ingredient of the formula

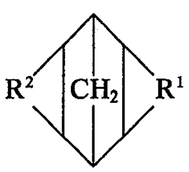

where $R^1$ is $-N-$ or $-N{=}N-N-$, $R^2$ is $-N{=}N-$ or $-NH-NH-$ and $R^3$ is

$R^3$ $R^3$

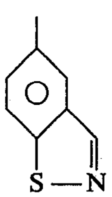

or phenyl which is substituted by halogen, fluoroalkyl of 1 or 2 carbon atoms, $NO_2$ or $C_1-C_3$-alkyl, the amount applied for the treatment being so chosen that less than 100 g of active ingredient is used per hectare of sown land.

2. A process as claimed in claim 1, wherein $R^3$ is 4-chlorophenyl or 4-bromophenyl.

**Revendications**

1. Procédé pour réguler la croissance des plantes et/ou pour améliorer l'utilisation, par les plantes, de l'eau disponible, caractérisé par le fait que l'on traite les graines des plantes avec un principe actif azoté, polycyclique, de formule

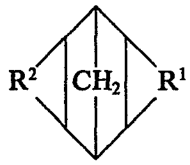

dans laquelle $R^1$ représente le reste 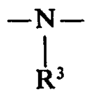 ou 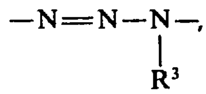, $R^2$ le reste $-N{=}N-$ ou $-NH-NH-$ et $R^3$ le reste

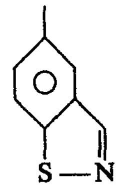

ou un reste phényle qui est substitué par halogène, fluoralkyle à 1 ou 2 atomes C, $NO_2$ ou alkyle en $C_1-C_3$, la dose d'emploi pour le traitement étant choisie pour que, pour la surface du sol ensemencée, il en résulte une quantité de principe actif inférieure à 100 g de principe actif par hectare.

2. Procédé selon la revendication 1, caractérisé par le fait que $R^3$ représente 4-chlorophényle ou 4-bromophényle.